# EUROPÄISCHE PATENTANMELDUNG

(11) **EP 0 692 471 A1**
(43) Veröffentlichungstag der Anmeldung: **17.01.1996**
(21) Anmeldenummer: 95110685.5
(22) Anmeldetag: 08.07.1995
(51) Int. Cl.: C07D 207/12

(54) **Verfahren zur Herstellung optisch aktiver 3-Hydroxy-Pyrrolidine mit hoher Enantiomerenreinheit**

(30) Priorität: 15.07.1994 DE 4425071
(71) Anmelder: Degussa Aktiengesellschaft, D-60311 Frankfurt (DE)
(72) Erfinder: Drauz, Karlheinz, Prof.Dr., D-63579 Freigericht (DE); Kottenhahn, Matthias, Dr., D-63579 Freigericht (DE); Kraft, Michael, D-63517 Rodenbach (DE); Schwarm, Michael Dr., D-63755 Alzenau (DE)

(57) **Zusammenfassung**

Bei bekannten Verfahren werden Pyrrolidine der allgemeinen Formel 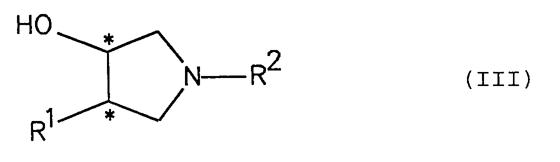
worin
- R¹: Wasserstoff oder OH ist,
- R²: Benzylrest, der am Aromaten ein oder mehrere Alkyl-, Alkoxy- und/oder Halogensubstituenten aufweisen kann, und
- *: ein Asymmetriezentrum ist und/oder sein kann,
durch Reduktion der entsprechenden enantiomerenreinen Pyrrolidindione mit entweder vergleichsweise teuren oder zum Teil sicherheitstechnisch oder unter Umweltschutzaspekten problematischen Reagenzien hergestellt.

Dadurch, daß zur Reduktion ein aktiviertes Alkaliborhydrid verwendet wird, bevorzugt NaBH₄, das mit Schwefelsäure aktiviert wird, werden die Verbindungen der Formel (III) in hoher Ausbeute und Enantiomerenreinheit in einem einfachen und kostengünstigem Verfahren erhalten.

## Beschreibung

Die Erfindung beschreibt ein neues Verfahren zur Herstellung von optisch aktiven 3-Hydroxy- oder 3,4-Dihydroxypyrrolidinen. Insbesondere betrifft die Erfindung ein Verfahren zur Herstellung optisch aktiver Pyrrolidine mit hoher Enantiomerenreinheit der allgemeinen Formel 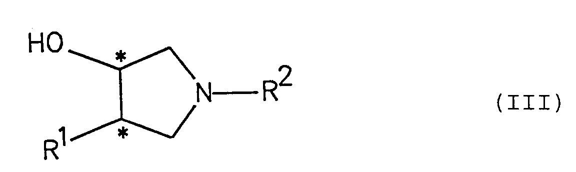
in der R¹ Wasserstoff oder eine Hydroxygruppe sein kann, R² ein Benzylrest ist, dessen aromatischer Rest ein oder mehrere Alkyl-, Alkoxy- und/oder Halogensubstituenten aufweisen kann und * ein Asymmetriezentrum ist, wenn das so gekennzeichnete Kohlenstoffatom eine Hydroxygruppe trägt, durch Reduktion enantiomerenreiner 2,5-Pyrrolidindione der allgemeinen Formel 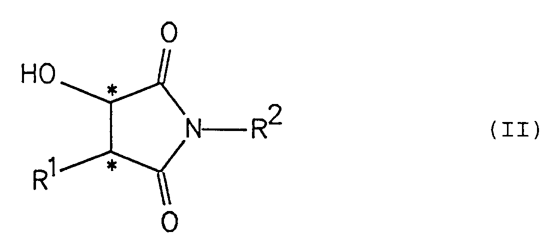
in der R¹, R² und * die bei Formel (III) angegebenen Bedeutungen haben.

Diese Verbindungen sind wichtige Zwischenprodukte in der chemischen Industrie. So sind Enantiomere des 3-Hydroxypyrrolidins, die aus Verbindungen der Formel III (R¹ = H) durch katalytische Hydrierung erhalten werden, Bausteine bei der Synthese von Carbapenem-Antibiotika (Chemotherapy 1991, 39 (Suppl. 3), 83 ff.; Tetrahedron Lett. 1984, 25 (26), 2793; JP 04036282 A2, 6. Feb. 1992; JP 03275685 A2, 06.12.1991; Korean J. Med. Chem. 1993, 3 (1), 72). Enantiomere des 1-Benzyl-3,4-dihydroxypyrrolidins werden bei der Synthese von optisch aktiven Phosphanen eingesetzt, die als Liganden von Metallkatalysatoren für asymmetrische Hydrierungen dienen (Chem. Ber. 1986, 119, 3326; DE 3446303 A1, 19. Juni 1986; DE 3403194 A1, 1. Aug. 1985; Bull. Chem. Soc. Jpn. 1984, 57 (3), 823).

Zur Herstellung dieser Verbindungen sind verschiedene Wege beschrieben worden. So sind optisch aktive 3-Hydroxypyrrolidine durch klassische (JP 05279326 A2, 26. Okt. 1993; JP 05279325 A2, 26. Okt. 1993; JP 05032620, 9. Feb. 1993; JP 04164066 A2, 9. Juni 1992; JP 04013659 A2, 17. Jan. 1992; JP 61063652 A2, 1. Apr. 1986) oder enzymatische Racematspaltung (JP 05227991 A2, 7. Sept. 1993; JP 04131093 A2, 1. Mai 1992; JP 01141600 A2, 2. Juni 1989) erhalten worden. Nachteil dieser Verfahren ist, daß die maximale Ausbeute nur 50 % beträgt, falls das unerwünschte Enantiomer nicht durch eine Racemisierung zurückgeführt werden kann.

Eine andere Möglichkeit ist die Decarboxylierung von optisch aktivem Hydroxyprolin (JP 05255204 A2, 5. Okt. 1993; Bioorg. Med. Chem. Lett. 1992, 2 (8), 827; Chem. Lett. 1986, (6), 893; JP 60023328 A2, 5. Feb. 1985). Dies ist insofern problematisch, als das geeignete optisch aktive Hydroxyprolin ein teures und in großen Mengen oft nicht verfügbares Ausgangsmaterial ist.

Weitere Verfahren beruhen auf der Cyclisierung geeigneter Butan- oder Buttersäurederivate, die dann gegebenenfalls noch weiter chemisch umgewandelt werden müssen (EP 452143 A2, 16. Okt. 1991; EP 431521 A1, 12. Juni 1991; EP 347818 A2, 27. Dez. 1989; JP 01045360 A2, 17. Feb. 1989, EP 269258 A2, 1. Juni 1988). Synthesen ausgehend von L-Glutaminsäure (Synth. Comm. 1986, 16 (14), 1815) bzw. N-substituierten 3-Pyrrolinen (J. Org. Chem. 1986, 51 (22), 4296) sind ebenfalls bekannt. Diese Verfahren bedingen eine vergleichsweise aufwendige Chemie oder gehen von nicht leicht erhältlichen Vorstufen aus.

Sehr gut geeignete und in großen Mengen leicht und billig erhältliche Edukte sind optisch aktive Äpfel- und Weinsäuren. Diese können mit Benzylamin relativ leicht zu den entsprechenden Imiden cyclisiert werden, die dann zu den optisch aktiven Hydroxypyrrolidinen reduziert und gegebenenfalls hydrogenolytisch debenzyliert werden können. Eine Gefahr stellt dabei allerdings die beschriebene Cyclisierung von Weinsäure mit Benzylamin in Xylol dar, da das gebildete Imid dabei gegen Ende der Reaktion "unter heftigem Aufsieden" auskristallisiert (Chem. Ber. 1986, 119, 3327). Die Reduktion der Imide zu den Hydroxypyrrolidinen ist zwar prinzipiell leicht, bisher aber nur mit vergleichsweise teuren und zum Teil sicherheitstechnisch oder unter Umweltschutzaspekten problematischen Reagentien durchführbar, z. B. Natriumborhydrid-Bortrifluorid-etherat (Chem. Ber. 1986, 119, 3327), Natriumaluminiumhydrid (JP 03200762 A2, 2. Sept. 1991), Na[AlH₂(OCH₂CH₂OMe)₂] (JP 01254657 A2, 11. Okt. 1989) oder Lithiumaluminiumhydrid (JP 01207266 A2, 21. Aug. 1989).

In Anbetracht des hierin dargelegten Standes der Technik ist es Aufgabe der Erfindung, ein weiteres Verfahren zur Herstellung von Verbindungen der eingangs erwähnten Art zur Verfügung zu stellen, das bei Einsatz leicht und billig erhältlicher Edukte die Gewinnung der angestrebten optisch aktiven Pyrrolidine in hoher Enantiomerenreinheit gestattet und gleichzeitig möglichst wenig aufwendig durchführbar ist.

Diese und weitere nicht näher genannte Probleme werden durch ein Verfahren der eingangs erwähnten Gattung mit den Merkmalen des kennzeichnenden Teils des Anspruchs 1 gelöst.

Dadurch, daß zur Reduktion der Verbindung der Formel (II) ein aktiviertes Alkaliborhydrid verwendet wird, wird eine einfach durchführbare und vor allem auch im technischen Maßstab gut handhabbare Reduktionsvariante zur Verfügung gestellt, die zudem eine erstaunlich hohe Enantiomerenreinheit der Produkte bedingt.

Die Reduktion der Imide der Formel II zu den entsprechenden optisch aktiven 1-substituierten-3-Hydroxy- oder 3,4-Dihydroxypyrrolidinen (Verbindungen der Formel III) verläuft im Rahmen der Erfindung gemäß dem folgenden Formelschema: 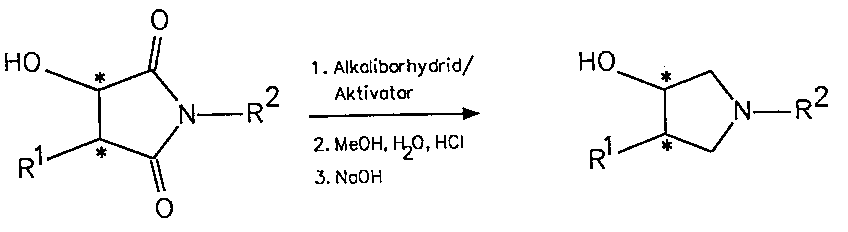
Erfindungsgemäß ist das zur Reduktion zu verwendende Alkaliborhydrid immer zu aktivieren. Bevorzugt hierfür einsetzbare Aktivatoren sind u. a. Jod, Chlorwasserstoff und/oder Schwefelsäure. Von den genannten Aktivatoren ist Schwefelsäure für die Erfindung ganz besonders bevorzugt.

Zu den mit Vorteil in der Erfindung einzusetzenden Alkaliborhydriden gehören Lithium-, Natrium- und Kaliumborhydrid. Hiervon sind Lithium- und Natriumborhydrid bevorzugt und Natriumborhydrid ist aufgrund seines geringen Preises besonders bevorzugt.

Die aufgezählten Reduktionssysteme sind einfach und, besonders auch in technischem Maßstab, vergleichsweise gefahrlos zu handhaben. Die nach der Reaktion verbleibenden Rückstände dieser Reagentien sind, verglichen mit den bisher benutzten Systemen (siehe oben), relativ problemlos zu entsorgen.

Der Rest R² ist wie bereits erwähnt eine Benzylgruppe, deren aromatischer Bestandteil ein oder mehrere Alkyl-, Alkoxy- und/oder Halogensubstituenten tragen kann. Bevorzugte Alkylgruppen sind C₁-C₄, auch verzweigt; bevorzugte Alkoxysubstituenten sind Methoxy, Ethoxy, Propoxy und Isopropoxy; bevorzugte Halogene sind F, Cl, Br, J.

In bevorzugter Ausführungsform der Erfindung ist der Rest R² eine Benzylgruppe und das verwendete Alkaliborhydrid NaBH₄. Das erfindungsgemäße Verfahren verläuft in diesem Fall gemäß der folgenden Gleichung: 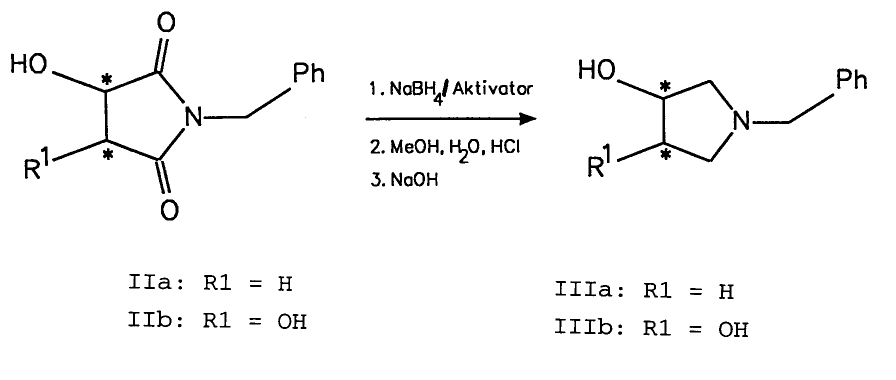
Das System Natriumborhydrid/Iod ist bereits bekannt für die Reduktion einer Vielzahl von Verbindungsklassen wie Aminosäuren (Tetrahedron Lett. 1992, 33, 38, 5517), Aminosäuren, N-Acyl-Aminosäuren und Aminosäureamide (J. Org. Chem. 1993, 58, 3568), Carbonsäuren (J. Org. Chem. 1991, 56, 5964), Ester, Amide und Nitrile (Tetrahedron 1992, 48 (22), 4623). Dagegen sind die Systeme Natriumborhydrid/Schwefelsäure und Natriumborhydrid/Chlorwasserstoff bisher nur für die Reduktion von Aminosäuren und N-Acylaminosäuren zu den entsprechenden Amino- und N-Alkylaminoalkoholen beschrieben worden (Tetrahedron Lett. 1992, 33 (38), 5517). Es wurde nun gefunden, daß diese Reduktionssysteme erfindungsgemäß besonders vorteilhaft zur Reduktion der Imide IIa und IIb zu den Pyrrolidinen IIIa und IIIb verwendbar sind, wobei sich überraschender- und vorteilhafterweise herausstellte, daß dabei die Stereochemie an den die Hydroxygruppe tragenden Chiralitätszentren vollständig erhalten blieb. Außerdem verlaufen die Reaktionen sauber und mit guter Rohausbeute.

Grundsätzlich ist es für das erfindungsgemäße Verfahren von Vorteil, wenn es in einem Lösungsmittel ausgeführt wird.

Das für die Reduktion eingesetzte Lösungsmittel sollte ein hinreichendes Lösungsvermögen für die eingesetzten Reaktionspartner besitzen. Besonders bevorzugt sind 1,2-Dimethoxyethan und Tetrahydrofuran, doch kommen prinzipiell auch andere Ether sowie Alkohole und Acetale in Frage.

Die Reaktion kann innerhalb eines weiten Temperaturbereichs (ca. -20 °C - Siedetemperatur des Lösungsmittels) durchgeführt werden, doch wird bevorzugt so verfahren, daß das Imid (beispielsweise die Verbindung IIa oder IIb) mit Alkaliborhydrid im Lösungsmittel vorgelegt, eine Lösung vom Aktivator (bevorzugt Iod oder Schwefelsäure) in diesem oder einem anderen geeigneten Lösungsmittel bei 0 - 40 °C, ggf. unter Kühlung, zugetropft und anschließend zur Vervollständigung der Reaktion mehrere Stunden bis maximal zur Siedetemperatur des verwendeten Lösungsmittels erhitzt wird. Pro Äquivalent Imid der Verbindung II werden 2 - 5 Äquivalente, bevorzugt 3 - 4 Äquivalente, Reduktionsmittel (bevorzugt Natriumborhydrid) eingesetzt. Vorteilhaft wird pro Äquivalent Alkaliborhydrid 1 Äquivalent Chlorwasserstoff oder 1/2 Äquivalent Iod (als I₂) oder Schwefelsäure als Aktivator zugesetzt.

Nach Beendigung der Reaktion wird das Reaktionsgemisch mit einem Alkohol und/oder Wasser hydrolysiert, das organische Lösungsmittel abdestilliert, der Rückstand im Wasser aufgenommen und mit Salzsäure oder einer anderen Säure sauer gestellt, einige Zeit gerührt, dann, bevorzugt mit Natronlauge, alkalisch gestellt und das Pyrrolidin der Formel III mit einem geeigneten organischen Lösungsmittel extrahiert. Es kann dann als Öl oder in kristalliner Form oder als Hydrochlorid isoliert und bei Bedarf durch Chromatographie oder Umkristallisation weiter gereinigt werden.

Die zur Reduktion einzusetzenden optisch aktiven 2,5-Pyrrolidindione (Formel II) werden in vorteilhafter Weiterbildung des erfindungsgemäßen Verfahrens durch Kondensation von optisch aktiven Verbindungen der Formel I mit einer Verbindung der Formel IV erhalten. Das folgende Formelschema dient zur Verdeutlichung: 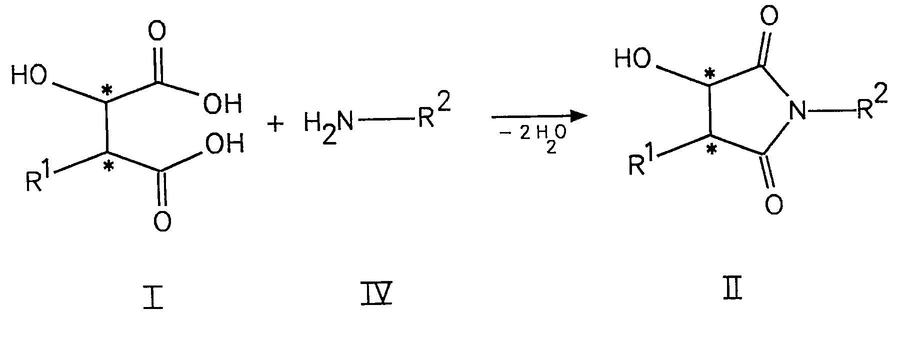
Hierin bedeuten R¹ Wasserstoff oder eine Hydroxygruppe, R² einen Benzylrest, dessen aromatischer Rest Alkyl-, Alkoxy- und/oder Halogensubstituenten aufweisen kann, und * ein Asymmetriezentrum, wenn das so gekennzeichnete Kohlenstoffatom eine Hydroxygruppe trägt.

Ganz besonders bevorzugt für die Erfindung ist Benzylamin als Verbindung IV.

Weiterhin besonders vorteilhaft wird daher eine optisch aktive Äpfelsäure (Ia) oder Weinsäure (Ib) mit Benzylamin zum optisch aktiven 1-Benzyl-3-hydroxy- (IIa) bzw. 1-Benzyl-3,4-dihydroxy-2,5-pyrrolidindion (IIb) cyclisiert. Dabei wurde überraschender- und vorteilhafterweise gefunden, daß die Reaktion in siedendem Cumol statt in Xylol nicht nur gefahrlos und gut kontrollierbar durchzuführen ist, sondern mit beendeter Wasserabscheidung auch praktisch quantitativ abläuft und das gebildete Imid nach Abdestillieren des Lösungsmittels ohne weitere Reinigung als Rohprodukt weiterverarbeitet werden kann, was sich günstig auf die Gesamtausbeute auswirkt.

Diese Reaktion ist nochmals im folgenden Schema dargestellt: 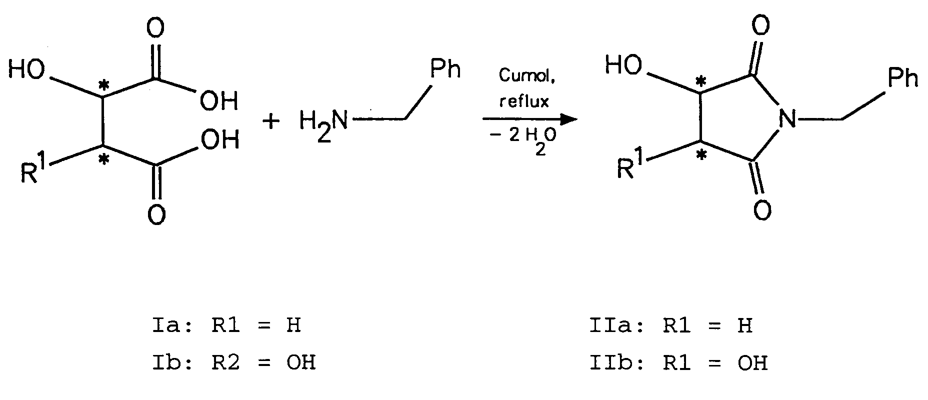
Insgesamt wird mit diesem neuen Verfahren ein einfacher und wirtschaftlicher Zugang zu optisch aktivem 1-Benzyl-3-hydroxy- oder 1-Benzyl-3,4-dihydroxypyrrolidin beschrieben. Es wird durch die folgenden Ausführungsbeispiele näher erläutert:

### Beispiel 1:

Ein Gemisch von 50,0 g (0,373 mol) (S)-Äpfelsäure (Ia), 41 ml (0,373 mol) Benzylamin und 250 ml Cumol wurde mit einer Innentemperatur von 155 °C 2,5 h am Wasserabscheider erhitzt, wobei 12 ml Wasser abgeschieden wurden. Beim Abkühlen bildete sich eine ölige Phase, die langsam kristallisierte. Danach wurde das Cumol abdekantiert und der Rückstand aus 220 ml EtOH kristallisiert. Es wurden 30,3 g (39,6 %) (S)-1-Benzyl-3-hydroxy-2,5-pyrrolidindion (IIa) in Form farbloser Kristalle isoliert.

Smp.: 110-113°C [α]D20: -57,3° (1,0, EtOH)

| | | | | |
|---|---|---|---|---|
| C11H11NO3 205,21 | ber. | C 64,38 | H 5,40 | N 6,83 |
| | gef. | C 64,03 | H 5,44 | N 6,96 |

Aus der Mutterlauge wurden durch Einengen weitere 8,7 g (11,4 %) IIa erhalten.

### Beispiel 2:

Zu einer Suspension von 25,6 g (0,125 mol) (S)-1-Benzyl-3-hydroxy-2,5-pyrrolidindion (IIa) und 19 g (0,5 mol) Natriumborhydrid in 150 ml DME wurde innerhalb von 3 h bei 25 - 33 °C eine Lösung von 63,4 g (0,25 mol) Iod in 200 ml DME getropft. Anschließend wurde über Nacht bei 70 °C gerührt. Nach Abkühlen wurden 40 ml MeOH zugesetzt, und der Ansatz wurde zur Trockne eingedampft. Der Rückstand wurde in 120 ml Wasser aufgenommen und mit 30 ml konz. Salzsäure und 150 ml Toluol über Nacht bei Raumtemperatur gerührt. Nach Alkalisieren mit 45 ml 50 %iger Natronlauge wurde die Toluolphase bei 70 °C abgetrennt und die Wasserphase mit 100 ml Toluol nachextrahiert. Die Toluolphasen wurden nach Trocknen über Natriumsulfat zur Trockne eingedampft, wobei 20,3 g (91,6 %) (S)-1-Benzyl-3-hydroxypyrrolidin (IIIa) als Öl erhalten wurden.

[α]D20: -3,6° (1,5, MeOH)

### Beispiel 3:

Zu einer Suspension von 25,6 g (0,125 mol) (S)-1-Benzyl-3-hydroxy-2,5-pyrrolidindion (IIa) und 19 g (0,5 mol) Natriumborhydrid in 150 ml DME wurde innerhalb von 1,5 h bei 35 °C eine Lösung von 13,4 ml (0,25 mol) Schwefelsäure in 40 ml DME getropft. Anschließend wurde 2 h bei 70 °C gerührt. Nach Abkühlen wurden 40 ml MeOH zugesetzt, und der Ansatz wurde zur Trockne eingedampft. Der Rückstand wurde in 120 ml Wasser aufgenommen und mit 30 ml konz. Salzsäure und 150 ml Toluol über Nacht bei Raumtemperatur gerührt. Nach Alkalisieren mit 45 ml 50 %iger Natronlauge wurde die Toluolphase bei 70 °C abgetrennt und die Wasserphase mit 100 ml Toluol nachextrahiert. Die Toluolphasen wurden nach Trocknen über Natriumsulfat zur Trockne eingedampft, wobei 22,7 g Öl erhalten wurden. Dieses wurde in 150 ml Toluol, 100 ml Wasser und 15 ml konz. Salzsäure aufgenommen und über Nacht bei Raumtemperatur gerührt. Nach Wiederholung der beschriebenen Aufarbeitung wurden 21,7 g (97,9 %) (S)-1-Benzyl-3-hydroxypyrrolidin (IIIa) als Öl erhalten.

[α]D20: -3,6° (1,5, MeOH)

### Beispiel 4:

Eine Mischung von 100 g (0,745 mol) (S)-Äpfelsäure (Ia), 81,4 ml (0,745 mol) Benzylamin und 500 ml Cumol wurde unter Stickstoff bei 153 °C Ölbadtemperatur 2,5 h am Wasserabscheider gekocht, wobei 25 ml Wasser abgetrennt wurden. Anschließend wurde zur Trockne einrotiert, in 250 ml DME aufgenommen und erneut zur Trockne einrotiert.

Es wurden 158 g (ca. 0,745 mol) rohes (S)-1-Benzyl-3-hydroxy-2,5-pyrrolidindion (IIa) erhalten. Dieses wurde in 800 ml DME gelöst, zu dem dann 85,1 g (2,25 mol) Natriumborhydrid gegeben wurden. Zur der entstandenen dicken Suspension wurde bei 20 - 32 °C innerhalb von 2,5 h eine Lösung von 60,3 ml (1,125 mol) Schwefelsäure in 200 ml DME getropft und anschließend 3 h bei 70 °C nachgerührt. Nach Abkühlen wurden 200 ml Methanol zugetropft, und der Ansatz wurde anschließend zur Trockne einrotiert. Der Rückstand wurde in 700 ml Wasser aufgenommen, unter Eiskühlung mit 180 ml konz. Salzsäure versetzt und 1 h bis zum Ende der Gasentwicklung gerührt. Anschließend wurden 270 ml Natronlauge und 1 l Toluol zugesetzt. Nach Erwärmen auf 70 °C wurde die Toluolphase abgetrennt und die Wasserphase mit 300 ml Toluol nachextrahiert. Nach Einrotieren der organischen Phase begann der Rückstand zu gasen, so daß er in 600 ml Wasser und 600 ml Toluol aufgenommen und mit 90 ml konz. Salzsäure über Nacht bei Raumtemperatur gerührt wurde. Anschließend wurde wieder basisch gestellt und wie oben extrahiert und einrotiert, wobei 131,3 g (99,4 %) rohes (S)-1-Benzyl-3-hydroxypyrrolidin (IIIa) erhalten wurden.

Die Struktur von IIIa wurde durch ein NMR-Spektrum bestätigt.

### Beispiel 5:

Zu einer Suspension von 22,1 g (0,1 mol) (3R,4R)-1-Benzyl-3,4-dihydroxy-2,5-pyrrolidindion (IIb) und 15,2 g (0,4 mol) Natriumborhydrid in 500 ml THF wurde innerhalb 1 h bei 12 - 15 °C eine Lösung von 50,7 g (0,2 mol) Iod in 200 ml THF getropft. Anschließend wurde 2 d unter Argon am Rückfluß erhitzt. Nach Abkühlen wurden 50 ml Methanol zugesetzt und nach Beendigung der Gasentwicklung zur Trockne eingedampft. Der Rückstand wurde in 60 ml Wasser aufgenommen und mit 15 ml konz. Salzsäure bis zur sauren Reaktion versetzt. Nach 30 min Rühren wurden 150 ml 20 % Kalilauge zugegeben und zweimal mit je 250 ml MTBE extrahiert. Nach Eindampfen verblieben 19,2 g farbloser, kristalliner Rückstand. Umkristallisation aus 60 ml Essigester ergab in 2 Fraktionen 10,4 g (53,9 %) (3S,4S)-1-Benzyl-3,4-dihydroxypyrrolidin (IIIb).

Smp.: 99-100,5 °C [α]D20: +32,7° (1,5, MeOH)

| | | | | |
|---|---|---|---|---|
| C11H15NO2 193,25 | ber. | C 68,37 | H 7,82 | N 7,25 |
| | gef. | C 68,10 | H 7,89 | N 7,42 |

### Beispiel 6:

Zu einer Suspension von 66,4 g (0,3 mol) (3R,4R)-1-Benzyl-3,4-dihydroxy-2,5-pyrrolidindion (IIb) und 45,6 g (1,2 mol) Natriumborhydrid in 400 ml 1,2-Dimethoxyethan (DME) wurde innerhalb von 2,5 h bei Raumtemperatur eine Lösung von 32,2 ml (0,6 mol) Schwefelsäure in 100 ml DME getropft. Anschließend wurde 4 h bei 75 °C gerührt. Nach Abkühlen wurden 75 ml MeOH zugesetzt und zur Trockne einrotiert. Der Rückstand wurde in 300 ml Wasser aufgenommen und mit 50 ml konz. Salzsäure versetzt. Anschließend wurde alkalisch gestellt und bei 65 °C mit zweimal 200 ml Toluol extrahiert. Der Extraktionsrückstand wurde in je 100 ml Wasser und Toluol aufgenommen, mit Salzsäure stark sauer eingestellt und über Nacht bei Raumtemperatur gerührt. Anschließend wurde mit 30 %iger Natronlauge alkalisch gestellt. Nach Zusatz von 200 ml Toluol wurde bei 65 °C die organische Phase abgetrennt. Die Wasserphase wurde nochmals mit 200 ml Methylenchlorid extrahiert. Nach Einrotieren der organischen Phasen verblieben 85 g Öl. Dieses wurde in 400 ml Essigester aufgenommen und bei 65 °C zweimal mit 80 ml Wasser gewaschen. Die Essigesterphase wurde nach Trocknen über Natriumsulfat einrotiert. Es verblieben 60 g Rückstand, die aus 150 ml Essigester umkristallisiert wurden. In zwei Fraktionen wurden 30,2 g (52,1 %) (3S,4S)-1-Benzyl-3,4-dihydroxypyrrolidin (IIIb) erhalten.

Smp.: 97-98°C [α]D20: +33,5° (1,5, MeOH)

| | | | | |
|---|---|---|---|---|
| C11H15NO2 193,25 | ber. | C 68,37 | H 7,82 | N 7,25 |
| | gef. | C 68,30 | H 7,98 | N 7,32' |

### Beispiel 7:

Zu einer Suspension von 37,8 g (1,0 mol) Natriumborhydrid und 51,3 g (0,25 mol) (S)-1-Benzyl-3-hydroxy-2,5-pyrrolidindion (IIa) in 300 ml DME wurden innerhalb von 2 h bei 15 - 20 °C 173 g einer Lösung von Chlorwasserstoff in DME (21,1 Gew.-% = 36,5 g (1,0 mol) HCl) getropft. Die Mischung wurde auf 70 °C erhitzt und nach Zusatz von weiteren 100 ml DME, 3 h bei 70 °C gerührt. Nach Abkühlen wurden 80 ml MeOH zugetropft, und der Ansatz wurde zur Trockne einrotiert. Der Rückstand wurde in 240 ml Wasser aufgenommen und nach Zusatz von 60 ml konz. Salzsäure bei Raumtemperatur bis zum Ende der Gasentwicklung gerührt. Nach Zusatz von 300 ml Toluol wurde die Mischung mit 100 ml 30 % Natronlauge alkalisch gestellt. Die Toluolphase wurde bei 70 °C abgetrennt, mit 100 ml Wasser und 30 ml konz. Salzsäure versetzt und über Nacht bei Raumtemperatur gerührt. Die Mischung wurde dann wieder mit 50 ml 30 % Natronlauge alkalisiert und die Toluolphase bei 70 °C abgetrennt. Nach Filtration wurde die Toluolphase zur Trockne einrotiert und ergab 44.8 g (praktisch quantitativ) (S)-1-Benzyl-3-hydroxypyrrolidin (IIIa) als nahezu farbloses Öl.

Die Struktur von IIIa wurde durch ein NMR-Spektrum bestätigt.

Weitere Vorteile und Einzelheiten der Erfindung ergeben sich aus den nachfolgenden Patentansprüchen.

## Patentansprüche

1. Verfahren zur Herstellung optisch aktiver Pyrrolidine mit hoher Enantiomerenreinheit der allgemeinen Formel 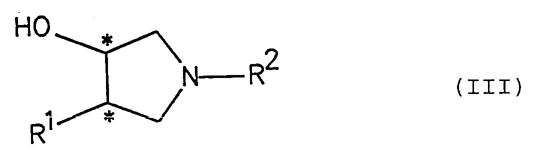 in der R¹ Wasserstoff oder eine Hydroxygruppe sein kann, R² ein Benzylrest ist, dessen aromatischer Rest einen oder mehrere Alkyl-, Alkoxy- und/oder Halogensubstituenten aufweisen kann, und * ein Asymmetriezentrum ist, wenn das so gekennzeichnete Kohlenstoffatom eine Hydroxygruppe trägt, durch Reduktion enantiomerenreiner 2,5-Pyrrolidindione der allgemeinen Formel 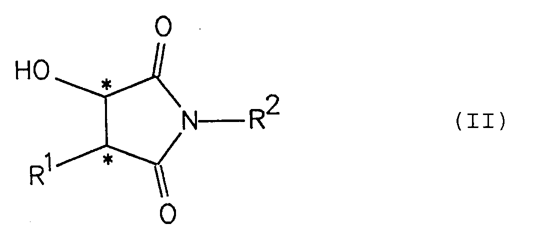 in der R¹, R² und * die bei Formel (III) angegebenen Bedeutungen haben,
**dadurch gekennzeichnet,**
daß zur Reduktion ein aktiviertes Alkaliborhydrid verwendet wird.

2. Verfahren nach Anspruch 1,
**dadurch gekennzeichnet,**
daß das Alkaliborhydrid mit Jod, Schwefelsäure oder Chlorwasserstoff aktiviert wird.

3. Verfahren nach Anspruch 1 oder 2,
**dadurch gekennzeichnet,**
daß als Alkaliborhydrid Natrium- oder Lithiumborhydrid eingesetzt wird.

4. Verfahren nach einem der vorhergehenden Ansprüche,
**dadurch gekennzeichnet,**
daß die Reaktion in einem Lösungsmittel, bevorzugt einem Ether, durchgeführt wird.

5. Verfahren nach Anspruch 4,
**dadurch gekennzeichnet,**
daß die Reaktion zwischen -20 °C und der Siedetemperatur des verwendeten Lösungsmittels durchgeführt wird.

6. Verfahren nach einem der vorhergehenden Ansprüche,
**dadurch gekennzeichnet,**
daß das Reaktionsgemisch nach beendeter Reduktion durch Zugabe eines Alkohols, dann Wasser und einer Säure hydrolysiert wird, anschließend alkalisiert wird, die optisch aktiven Pyrrolidine der allgemeinen Formel III mit einem organischen Lösungsmittel extrahiert werden und anschließend durch Eindampfen oder Kristallisation oder Fällung als Hydrochlorid isoliert werden.

7. Verfahren nach einem der vorhergehenden Ansprüche,
**dadurch gekennzeichnet,**
daß die zu reduzierenden optisch aktiven 2,5-Pyrrolidindione der allgemeinen Formel II durch Kondensation von optisch aktiver Äpfelsäure oder Weinsäure mit Benzylamin in Cumol hergestellt werden, wobei das entstehende Reaktionswasser azeotrop abdestilliert wird.

8. Verfahren nach Anspruch 7,
**dadurch gekennzeichnet,**
daß die optisch aktiven 2,5-Pyrrolidindione der allgemeinen Formel II für die Reduktion als Rohprodukte eingesetzt werden.
